(19) Europäisches Patentamt
European Patent Office
Office européen des brevets

(11) Veröffentlichungsnummer: **0 063 337**
**A1**

(12) # EUROPÄISCHE PATENTANMELDUNG

(21) Anmeldenummer: 82103072.3

(22) Anmeldetag: 09.04.82

(51) Int. Cl.³: C 07 D 307/935
A 61 K 31/34

(30) Priorität: 14.04.81 HU 96781

(43) Veröffentlichungstag der Anmeldung: 27.10.82 Patentblatt 82/43

(84) Benannte Vertragsstaaten: BE CH DE FR GB IT LI NL SE

(71) Anmelder: CHINOIN Gyógyszer és Vegyészeti Termékek Gyára RT.
Tó utca 1-5
H-1045 Budapest V(HU)

(72) Erfinder: Szántay, Csaba, Dr. Dipl.Ing.Chem.
Zsombolyai u.8
H-1113 Budapest(HU)

(72) Erfinder: Novák, Lajos, Dr. Dipl.Ing.Chem.
Szolyva u 2/b
H-1126 Budapest(HU)

(72) Erfinder: Aszódi, József, Dipl.Ing.Chem.
Stromfeld u. 25
H-2132 Göd-felsö(HU)

(72) Erfinder: Rohály, János, Dr. Dipl.Ing.Chem.
Gellérthegy u. 27
H-1016 Budapest(HU)

(72) Erfinder: Simonidesz, Vilmos, Dr. Dipl.Ing.Chem.
Fenyö u. 13
H-1016 Budapest(HU)

(72) Erfinder: Ivanics, József, Dipl.Ing.Chem.
Elem u.20
H-1045 Budapest(HU)

(72) Erfinder: Kovács, Gábor, Dr. Dipl.Ing.Chem.
Róna Park 4
H-1142 Budapest(HU)

(72) Erfinder: Virág, Sándor, Dr.
Sallai I.u.29/a
H-1136 Budapest(HU)

(72) Erfinder: Körmöczy, Péter, Dr.
Uri u.33
H-1014 Budapest(HU)

(72) Erfinder: Stadler, István, Dr.
Népstadion u.18
H-1143 Budapest(HU)

(74) Vertreter: Lotterhos, Hans Walter, Dr.-Ing.
Lichtensteinstrasse 3
D-6000 Frankfurt am Main 1(DE)

(54) **13-Oxa-prostacyclin-Derivate, Verfahren zu deren Herstellung sowie diese Verbindungen enthaltende pharmazeutische Präparate.**

(57) Die Erfindung betrifft 3-Oxa-prostacyclin-Derivate der Formel I

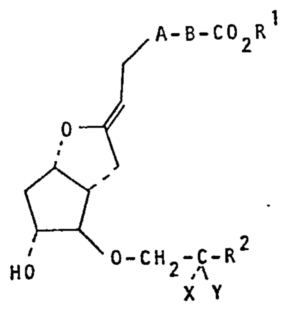

(I)

($R^1$ = H, pharmazeutisch verträgliches Kation, geradkettiges oder verzweigtes $C_{1-5}$-Alkyl,
$R^2$ = geradkettiges oder verzweigtes $C_{4-9}$-Alkyl oder monosubstituiertes Phenyloxymethyl,
-A-B- = $-CH_2-CH_2-$, E- oder Z- $-CH=CH-$ oder $-C\equiv C-$,
eines der symbole X und Y = Wasserstoffatom, Methyl oder Äthyl und das andere = Hydroxy, Tetrahydropyranyloxy, 1-Äthoxy-äthoxy oder Tri-$C_{1-5}$-alkylsilyloxy oder X und Y gemeinsam eine $-O-CH_2-CH_2-O-$Gruppe)
und ein Verfahren zu deren Herstellung aus Verbindungen der Formel II

./...

EP 0 063 337 A1

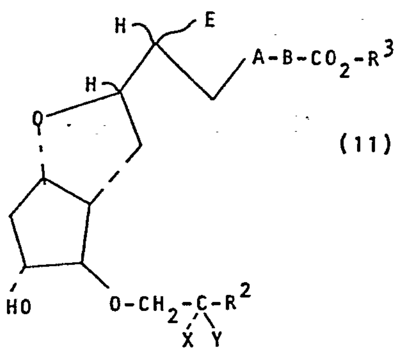

(11) ,

($R^3$ = geradkettiges oder verzweigtes $C_{1-5}$-Alkyl oder Wasserstoff und
E = Jod oder Brom

Die Verbindungen der Formel I besitzen wertvolle blutplättchenaggregationshemmende Eigenschaften und können in der Therapie verwendet werden.

13-Oxa-prostacyclin-Derivate, Verfahren zu deren Herstellung sowie diese Verbindungen enthaltende pharmazeutische Präparate

Die Erfindung betrifft neue, optisch aktive und racemische 13-Oxa-prostacyclin-Derivate der allgemeinen Formel I

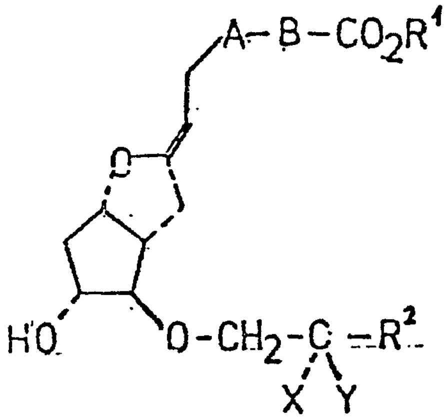

(I) ,

worin

$R^1$ Wasserstoff, ein pharmazeutisch verträgliches Kation oder geradkettiges oder verzweigtes $C_{1-5}$-Alkyl,

$R^2$ geradkettiges oder verzweigtes $C_{4-9}$-Alkyl oder monosubstituiertes Phenyloxymethyl,

-A-B- $-CH_2-CH_2-$, E- oder Z- -CH=CH- oder -C≡C-,

eines der Symbole X und Y Wasserstoff, Methyl oder Äthyl und das andere Hydroxy, Tetrahydropyranyloxy, 1-Äthoxy-äthoxy oder Tri-$C_{1-5}$-alkylsilyloxy bedeuten oder

X und Y gemeinsam $-O-CH_2-CH_2-O-$ bilden.

Die biologisch aktiven, neuen Verbindungen der Erfindung der allgemeinen Formel I können als stabilisierte Analoga des biologisch äußerst wirksamen Prostacyclins angesehen werden. Das Prostacyclin wurde zum ersten Mal im Jahre 1976 beschrieben (S.Moncada und Mitarbeiter: Nature 263, 663). Die Struktur dieser Verbindung wurde bald aufgeklärt (R.A.Johason und Mitarbeiter: Prostaglandines, 12, 915 (1976)). Das Prostacyclin ist ein Zwischenprodukt des Arachidonsäuremetabolismus

und übt bereits in einer sehr geringen Menge äußerst starke und wertvolle pharmazeutische Wirkungen aus. Von diesen Wirkungen scheint die blutplättchenaggregationshemmende und die vasodilatatorische Wirkung am wichtigsten zu sein. Mit Hilfe dieser Wirkungen kann das Prostacyclin als Arzneimittel zur Verhütung und Behandlung der heutzutage so häufigen Kreislaufanomalien - wie die häufige Arteriosklerose und Thrombose - Verwendung finden. In der Literatur sind über die klinische Anwendung des Prostacyclins mehrere Artikel erschienen (z.B. J.R.Vane und S. Bergström: Prostacyclin, Raven Press, New York 1979; S.Moncada und J.R. Vane: Advances in prostaglandin and thromboxane Research, Vol. 6. 43 - 60 Raven Press, New York, 1980).

Das wichtigste Hindernis bei der praktischen Anwendung des Prostacyclins stellt dessen äußerst große Instabilität dar. Die Halbwertzeit des Prostacyclins beträgt im Blut bei Körpertemperatur (37°C) etwa 2 - 3 Minuten (S.Moncada und J.R.Vane: Advances in prostglandin and thromboxane Research Vol. 6, 43). Die ausgezeichnete Wirkungsstärke des Prostacyclins ist mit einer sehr kurzen Wirkungsdauer verbunden.

Obwohl der chemisch empfindlichste Punkt des Prostacyclinmoleküls die Enolätherstruktur ($C_{5-9}$) ist - in Gegenwart einer äußeren oder inneren Protonenquelle zersetzt sich das Molekül schnell unter Bildung von 6-Oxo-$PGF_{2\alpha}$ - ist dessen Metabolismus außergewöhnlich schnell. Im Organismus wird das Prostacyclin durch das Prostaglandin-dehydrogenase-enzymsystem unter Oxydation der $C_{13-20}$-Struktureneinheit rasch deaktiviert. Das Prostacyclin ist in Form der freien Säure instabil, zu den pharmakologischen und klinischen Untersuchungen werden dessen Salze und Ester verwendet.

Ein Weg zur Stabilisierung des Prostacyclins besteht in der Durchführung von strukturellen Änderungen am Molekül, die die Wirkung des Prostaglandin-dehydrogenase-enzymsystems herabsetzen.

Die neuen Verbindungen der Erfindung der allgemeinen Formel I enthalten an Stelle der 13,14-C-C-Doppelbindung ein Sauerstoffatom und eine Methylengruppe. Mit Rücksicht darauf, daß der Angriffspunkt des Dehydrogenase-enzymsystems die Stellung 15 und 13-14 ist, kann wegen der

erfindungsgemäßen Änderung das Molekül nur in geringerem Maße ein Substrat des Prostacyclin-dehydrogenase-enzymsystems sein, wobei die wertvolle pharmakologische Wirksamkeit des Prostacyclins erhalten bleibt.

Die neuen Verbindungen der allgemeinen Formel I hemmen an aus männlichem Blut isoliertem, an Blutplättchen reichem Plasma die durch $1x10^{-6}$ Mol/ml ADP hervorgerufene Aggregation in einer Konzentration von 20 -150 mg/ml ($LD_{50}$) - nach der Methode von Born gemessen - was einer 1/10 - 1/50 $PGI_2$-Wirkung entspricht.

An aus Kaninchenblut isoliertem, an Blutplättchen reichem Plasma kann eine 50 %ige Hemmung der durch $1x10^{-4}$ Mol/ml hervorgerufenen Aggregation bei Anwendung der Verbindungen der allgemeinen Formel I in Salzform mit einer Konzentration von 100 - 1000 mg/ml erreicht werden.

Die Verbindungen der allgemeinen Formel I senken den systologischen und peripheren arteriellen Blutdruck an mit Pentobarbitural narkotisierten Katzen. Die Aktivität beträgt 1/5 - 1/100 der Wirkungsstärke von Prostacyclin.

Wie das $PGI_2$, ziehen beide Verbindungen die isolierte Kaninchen-lungenarterie und die isolierte Meerschweinchen-trachea zusammen, diese Aktivität ist etwas geringer als die des Prostacyclins.

Diese Wirkungen weisen eindeutig auf einen Dosis-Wirkungs-Zusammenhang hin.

Die Erfindung betrifft auch ein Verfahren zur Herstellung der Verbindungen der allgemeinen Formel I aus den Verbindungen der allgemeinen Formel II

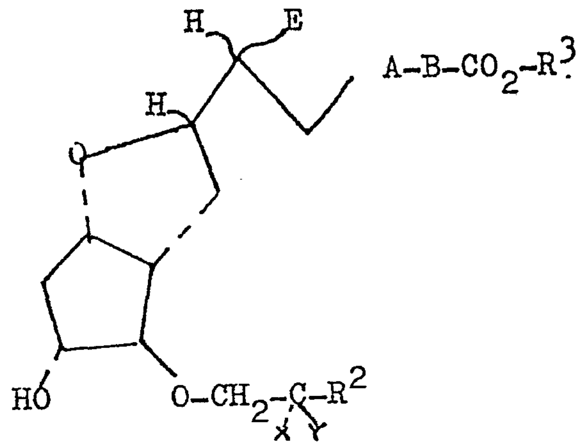

(II) ,

worin

$R^3$ geradkettiges oder verzweigtes $C_{1-5}$-Alkyl oder Wasserstoff und

E Jod oder Brom bedeuten und

$R^2$, -A-B-, X und Y die oben angegebene Bedeutung haben.

Die Umsetzung kann mit Hilfe einer organischen oder anorganischen Base - vorteilhaft mit 1,5-Diazabicyclo[4.3.0]-non-5-en - in einem wasserfreien aprotischen Lösungsmittel - vorteilhaft Toluol - oder in einem Alkohol oder in Abwesenheit eines Lösungsmittels durchgeführt werden. Die Reaktionstemperatur beträgt 25-150°C, vorzugsweise 100-120°C.

Die Verbindungen der allgemeinen Formel I können aus dem Reaktionsgemisch durch Waschen, Trocknen und Abdestillieren des Lösungsmittels isoliert werden. Die so erhaltenen Verbindungen der allgemeinen Formel I werden in einem Reinheitsgrad erhalten, daß sie nicht weiter gereinigt werden müssen. Die Verbindungen der allgemeinen Formel I, in denen $R^1$ nicht Wasserstoff ist, können nötigenfalls durch Säulen- oder Dünnschichtchromatographie gereinigt werden.

Die Verbindungen der allgemeinen Formel II können aus den neuen Verbindungen der allgemeinen Formel III

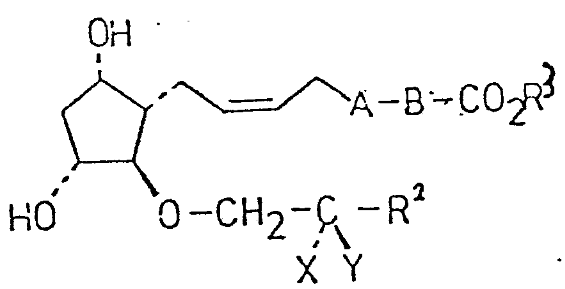

(III),

worin
$R^3$ Wasserstoff oder $C_{1-5}$-Alkyl ist und
$R^2$, -A-B-, X und Y die oben angegebene Bedeutung haben, hergestellt werden.

Die Cyclisierung kann mit Hilfe eines Halogenierungsmittels - vorteilhaft Jod - durchgeführt werden. Die Umsetzung wird in Gegenwart einer nicht zu starken Base - vorteilhaft Kaliumcarbonat oder Natriumbicarbonat - durchgeführt. Die Reaktionstemperatur liegt vorteilhaft zwischen etwa 0°C und 30°C. Als Reaktionsmedium können organische Lösungsmittel - insbesondere Dichlormethan oder Chloroform - dienen. Das aus dem Reaktionsgemisch durch Extraktion isolierte Rohprodukt kann durch Chromatographie gereinigt werden.

Bei Anwendung von Jod entsteht das Exo-Isomer der allgemeinen Formel IIa

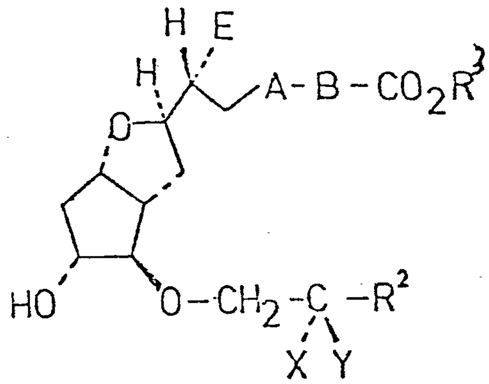

(IIa)

in einer Menge von über 95 %; das in einer Menge von etwa 4 % gebildete Endo-Isomer der allgemeinen Formel IIb

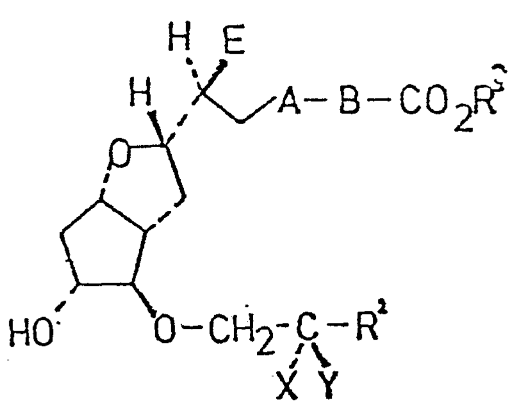

(IIb)

braucht nicht abgetrennt zu werden.

Bei der Anwendung von Bromierungsmitteln beträgt die Menge des gebildeten Endo-Isomers der allgemeinen Formel IIb etwa 10 %. In dem Fall wird das Endo-Isomer durch Säulenchromatographie abgetrennt, und zur Herstellung der Prostacyclinderivate der allgemeinen Formel I werden die Exo-Isomere verwendet.

Die Verbindungen der allgemeinen Formel III können durch Umsetzung von bekannten Verbindungen der allgemeinen Formel IV

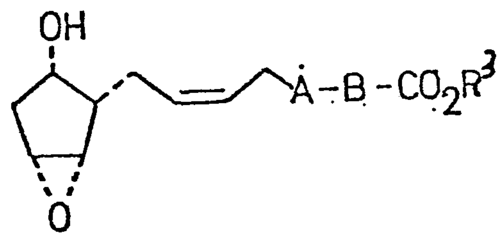

(IV)

(L.Novák, J.Rohály, M.Kajtár und Cs.Szántay: Acta Chim. Acad.Sci.Hung. 102, 91 (1979))

mit den ebenfalls bekannten Verbindungen der allgemeinen Formel V

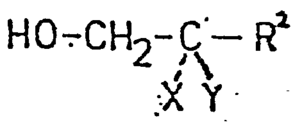

(V)

(L.Novák, J.Aszódi, P.Kolonits und Cs.Szántay: Tetrahedron Letters 1981, unter Publikation)

worin

$R^2$, $R^3$, X, Y und -A-B- die oben angegebene Bedeutung haben

hergestellt werden. Aus der Verbindung der allgemeinen Formel V wird zuerst mit einem Alkalimetall (z.B. Natrium) ein Alkoholat gebildet, das in einem wasserfreien Lösungsmittel oder in Abwesenheit eines Lösungsmittels mit einer Verbindung der allgemeinen Formel IV umgesetzt wird.

Die so erhaltenen Verbindungen der allgemeinen Formel III können von den Nebenprodukten durch Säulenchromatographie abgetrennt werden.

Die Bezeichnung "pharmazeutisch verträgliches Kation" bezieht sich auf solche ein-, zwei- oder dreiwertige positive Kationen, die im lebenden Organismus in den den erfindungsgemäßen Verbindungen entsprechenden Dosen keine unerwünschten Nebenwirkungen ausüben. Einige Beispiele für pharmazeutisch verträgliche Kationen sind: Alkalimetallkationen (z.B. Natrium, Kalium, Lithium), Erdalkalimetallkationen (z.B. Calcium, Magnesium), Aluminiumkationen, Ammoniumkationen oder aus verschiedenen organischen Aminen ableitbare ein- oder mehrwertige Ammoniumionen (z.B. tris-(Hydroxymethyl)-ammoniumionen).

Unter der Bezeichnung "geradkettiges oder verzweigtes $C_{1-5}$-Alkyl" sind Methyl, Äthyl, n-Propyl, Isopropyl, n-Butyl, Isobutyl, sec. Butyl und tert. Butyl sowie die verschiedenen Amylgruppen zu verstehen. Unter "geradkettigen oder verzweigten $C_{4-9}$-Alkylen" sind die verschiedenen Butyl-, Amyl-, Hexyl-, Heptyl-, Octyl- und Nonylgruppen zu verstehen.

Der Phenylring der Phenyloxymethylgruppe kann in irgend einer Stellung Halogen oder Trifluormethyl tragen.

Die drei $C_{1-5}$-Alkylsubstituenten der Trialkylsilylgruppe können identisch oder verschieden sein und die für die oben angegebenen $C_{1-5}$-Alkylgruppen angegebene Bedeutung haben.

Unter der Bezeichnung "organische Base" sind ein oder mehrere Stickstoffatome enthaltende, lineare, verzweigte oder cyclische Alkylamine zu verstehen (vorteilhaft 1,5-Diazabicyclo[4.3.0]non5-en oder Triäthylamin). Die "anorganischen Basen" können aus Alkalimetallen oder Erdalkalimetallen herleitbar sein, wie z.B. Alkoholate, Hydroxyde, Carbonate, Bicarbonate usw. .

Die bei dem erfindungsgemäßen Verfahren verwendbaren Alkohole enthalten vorzugsweise 1-5 Kohlenstoffatome. Als aprotische Lösungsmittel können vorteilhaft aromatische Kohlenwasserstoffe (wie Benzol, Toluol) oder halogenierte Kohlenwasserstoffe (wie Chloroform, Dichlormethan usw.) eingesetzt werden.

Als Halogenierungsmittel können in erster Linie elementare Halogene (wie Jod oder Brom) dienen. Zu diesem Zweck können jedoch auch aus der organischen Chemie bekannte andere Halogenierungsmittel eingesetzt werden (z.B. N-Brom-succinimid oder Interhalogene).

Gegenstand der Erfindung sind weiterhin pharmazeutische Präparate, die als Wirkstoff eine oder mehrere Verbindungen der allgemeinen Formel I und geeignete, inerte, übliche pharmazeutische Träger enthalten.

Weitere Einzelheiten der vorliegenden Erfindung sind den nachstehenden Beispielen zu entnehmen, ohne den Schutzumfang auf diese Beispiele einzuschränken.

Beispiel 1

Herstellung des 13-Oxa-13,14-dihydro-$PGI_2$-methylesters

A) Herstellung des 13-Oxa-13,14-dihydro-$PGF_{2\alpha}$-methylesters

Zu 8,76 g (66,3 mM) (-)-1,2-(S)-Heptandiol gibt man 0,3 g (13,2 Mg-atome) Natrium und rührt das Gemisch so lange auf einem Ölbad (50°C), bis das Natrium in Lösung geht. Der erhaltenen Lösung wird 1,0 g (4,4 mM) (-)-7-(5-Hydroxy-2,3-α-epoxy-cyclopenthyl)-5-(Z)-heptensäure zugefügt, das Reaktionsgemisch auf einem Ölbad bei 100 - 110°C 6 Stunden gerührt und nach dem Abkühlen in Wasser gegossen. Die wäßrige Lösung wird mit Äther extrahiert, die wäßrige Phase mit 5 %iger Salzsäure angesäuert (pH etwa 2) und mit Äther extrahiert. Der erhaltene Ätherextrakt wird mit gesättigter Natriumchloridlösung gewaschen, über Magnesiumsulfat getrocknet und unter vermindertem Druck eingeengt. Die als Rückstand erhaltene rohe Säure wird bei 0°C mit einer ätherischen Diazomethanlösung verestert und der erhaltene rohe Ester von dem als Nebenprodukt gebildeten Methyl-7-[2,5-dihydroxy-3-(-3-hydroxy-heptyl-oxy)-5-(Z)-heptanoat] durch Säulenchromatographie abgetrennt (Kieselgel G; 4 : 2 Chloroform-Aceton-Gemisch). Es werden 0,136 g des 13-Oxa-13,14-dihydro-$PGF_{2\alpha}$-methylesters erhalten, Ausbeute 18,0 %.

$R_f$ = 0,6 (7 : 3 : 0,1 Chloroform-Aceton-Essigsäure-Gemisch).

IR(NaCl): 3300 (OH), 1725 (CO), 1460, 1440, 1360, 1240, 1100, 1040 $cm^{-1}$.

Ms: $M^+$ 372 (13), m/e 354 (3), 336 (5), 222 (50), 209 (11), 196 (14), 191 (17), 180 (11), 143 (13), 140 (46), 130 (11), 129 (4), 121 (9), 119 (11), 115 (10), 109 (14), 105 (11), 99 (16), 97 (20), 93 (15), 91 (14), 83 (25), 81 (32), 80 (30), 79 (25), 67 (34), 55 (100).

B) Herstellung des 5-Jod-13-oxa-13,14-dihydro-$PGI_1$-methylesters

Einer Suspension von 0,098 g (0,26 mM) 13-Oxa-13,14-dihydro-$PGF_{2\alpha}$-methylester, 0,108 h (0,74 mM) Calciumcarbonat, 12 ml Methylenchlorid und 0,16 ml Wasser gibt man 0,08 g (0,32 mM) Jod unter Rühren zu und rührt das Reaktionsgemisch bei Raumtemperatur 3 Stunden. Danach setzt man dem Reaktionsgemisch Natriumthiosulfat so lange zu, bis die Farbe des Jods verschwindet. Die organische Phase wird abgetrennt, getrocknet und unter vermindertem Druck eingeengt und der erhaltene Rückstand durch Säulenchromatographie gereinigt (Kieselgel G; 4 : 1 Chloroform-Aceton-Gemisch).

Es werden 0,078 g des 5-Jod-13-oxa-13,14-dihydro-$PGI_1$-methylesters erhalten, Ausbeute 58 %.

$R_f$ = 0,46 (7 : 3 Chloroform-Aceton-Gemisch).

Ms: $M^+$ 498 (> 1 %), m/e 480 (1), 398 (2), 371 (14), 352 (6), 257 (15), 212 (25), 196 (12), 158 (13), 143 (18), 142 (15), 127 (29), 125 (20), 115 (8), 111 (19), 97 (24), 95 (18), 79 (24), 55 (100).

C) Herstellung des 13-Oxa-13,14-dihydro-$PGI_2$-methylesters

Einer Lösung von 0,076 g (0,152 mM) 5-Jod-13-oxa-13,14-dihydro-$PGI_1$-methylester und 10 ml wasserfreiem Toluol setzt man 0,094 mg (0,76 mM) 1,5-Diazabicyclo[4.3.0]non-5-en zu, worauf die Lösung eine Stunde zum Sieden erhitzt wird. Nach dem Abkühlen wird das Reaktionsgemisch mit einer 2 %igen Kaliumcarbonatlösung ausgeschüttelt, über Magnesiumsulfat getrocknet und unter vermindertem Druck eingeengt. Es werden 0,054 mg 13-Oxa-13,14-dihydro-$PGI_2$-methylester erhalten, Ausbeute 95,7 %.

$R_f$ = 0,63 (auf einer mit Trimethylamin behandelten Dünnschichtchromatographieplatte; Kaliumcarbonat enthaltendes 7 : 3 Chloroform-Aceton-Gemisch).

Ms: $M^+$ 370 (15), m/e 257 (32), 239 (26), 238 (18), 213 (13), 210 (14), 207 (16), 196 (32), 195 (32), 161 (14), 143 (56), 125 (25), 121 (15), 115 (45), 113 (32), 111 (32), 107 (14), 99 (32), 97 (56), 96 (24), 95 (32), 83 (27), 81 (32), 71 (18), 73 (14), 71 (14), 67 (18), 59 (17), 57 (18), 55 (100), 43 (32), 41 (32), 29 (26).

Beispiel 2

Herstellung des 15-Methyl-13-oxa-13,14-dihydro-$PGI_2$-methylesters und des 15-Epi-15-methyl-13-oxa-13,14-dihydro-$PGI_2$-methylesters

A) Herstellung des 15-Methyl-13-oxa-13,14-dihydro-$PGF_{2\alpha}$-methylesters und des 15-Epi-15-methyl-13-oxa-13,14-dihydro-$PGF_{2\alpha}$-methylesters

0,61 g (26,7 Mg-atome) Natrium löst man in 13,0 g (88,9 mM) (±)-2-Methyl-1,2-heptandiol bei 50°C, worauf man 2,0 g (8,89 mM) (-)-7-(5-Hydroxy-2,3-α-epoxy-cyclopentyl)-5-(Z)-heptensäure zugibt und das Reaktionsgemisch bei 100 - 110°C 6 Stunden rührt. Das erhaltene Reaktionsgemisch wird abgekühlt, in Wasser gegossen und mit Äther extrahiert. Die wäßrige Phase wird mit 5 %iger Salzsäure angesäuert und mit Äther extrahiert. Die letztere ätherische Lösung wird mit gesättigter Natriumchloridlösung gewaschen, über Magnesiumsulfat getrocknet und unter vermindertem Druck eingeengt. Die als Rückstand erhaltene rohe Säure wird mit einer ätherischen Diazomethanlösung (Konzentration 12 g/l bei 0°C) in den Methylester überführt. Der erhaltene rohe Ester wird von dem als Nebenprodukt gebildeten Methyl-7-[2,5-Dihydroxy-3-(3-hydroxy-3-methylheptyloxy)-5-(Z)-heptanoat] durch Säulenchromatographie abgetrennt (Kieselgel G; 4 : 1 Chloroform-Aceton-Gemisch). Ausbeute: 0,672 g (20,4 %).

$R_f$ = 0,59 (7 : 3 : 0,1 Chloroform-Aceton-Essigsäure-Gemisch).

IR(NaCl): 3350 (OH), 1730 (CO), 1460, 1440, 1360, 1240, 1090, 1040 $cm^{-1}$.

$^1$H-NMR($CDCl_3$): 0,89 (3H, t, J=6 Hz, $CH_3$), 1,12 und 1,15 (3H, 2s, $CH_3$), 1,3 (8H, mc, $4xCH_2$), 3,3 - 3,6 [2H, m, 2xABq, $(AB)_I$= 3,33 ppm, 3,50 ppm, $J_{AB}$= 9,5 Hz; $(AB)_{II}$= 3,38 ppm, 3,45 ppm, $J_{AB}$= 9,5 Hz, $OCH_2$], 3,6 - 3,8 (6H, m+s, 3,67 ppm, $OCH_3$, 3xOCH), 545 (2H, mc, CH=CH).

Ms: $M^+$ 386 (< 1 %), m/e 368 (3), 315 (20), 297 (5), 279 (7), 272 (38), 236 (23), 222 (78), 206 (30), 196 (35), 191 (36), 180 (20), 158 (16), 140 (100), 115 (65), 109 (50), 81 (62), 83 (63), 55 (90).

B) Herstellung des 5-Jod-15-methyl-13-oxa-13,14-dihydro-$PGI_1$-methylesters und des 15-Epi-15-methyl-13-oxa-13,14-dihydro-$PGI_1$-methylesters

0,231 g (0,6 mM) des nach Absatz A) hergestellten, aus 15-Methyl-13-oxa-13,14-dihydro-$PGF_{2}\alpha$-methylester und 15-Epi-15-methyl-13-oxa-13,14-dihydro-$PGF_{2}\alpha$-methylester bestehenden Gemisches und 0,249 g (1,8 mM) Kaliumcarbonat suspendiert man in 10 ml Methylenchlorid und 0,4 ml Wasser, gibt der erhaltenen Suspension 0,183 g (0,72 mM) Jod zu, worauf man das Gemisch bei Raumtemperatur 3 Stunden rührt. Dem erhaltenen Reaktionsgemisch wird Natriumthiosulfat so lange zugegeben, bis die Jodfarbe verschwindet. Sodann wird die organische Phase abgetrennt, über Magnesiumsulfat getrocknet und unter vermindertem Druck eingeengt. Der Rückstand wird durch Säulenchromatographie gereinigt (Kiesel G; 4 : 1 Chloroform-Aceton-Gemisch). Ausbeute: 0,157 g, 61 %. $R_f$ = 0,53 (7 : 3 Chloroform-Aceton).

Ms: $M^+$ 512 (< 1), m/e 476 (1), 397 (2), 376 (1), 367 (2), 347 (2), 331 (13), 253 (14), 239 (5), 222 (5), 205 (13), 191 (7), 142 (100), 127 (65), 115 (9), 111 (24), 109 (12), 95 (15), 83 (19), 81 (19), 55 (44).

C) Herstellung des 15-Methyl-13-oxa-13,14-dihydro-$PGI_2$methylesters und des 15-Epi-15-methyl-13-oxa-13,14-dihydro-$PGI_2$-methylesters

0,105 g (0,2 mM) des nach Absatz B) hergestellten, aus 5-Jod-15-methyl-13-oxa-13,14-dihydro-$PGI_1$-methylester und 15-Epi-15-methyl-13-oxa-13,14-dihydro-$PGI_1$-methylester bestehenden Gemisches und 0,128 g (1 mM) 1,5-Diazabicyclo[4.3.0]non5-en werden in 13 ml wasserfreiem Toluol eine Stunde zum Sieden erhitzt. Das erhaltene Reaktionsgemisch wird abgekühlt, mit einer 2 %igen Kaliumcarbonatlösung ausgeschüttelt, über Magnesiumsulfat getrocknet und unter vermindertem Druck eingeengt. Ausbeute 0,077 g, (97,3 %).

$R_f$ = 0,75 (auf einer, mit Triäthylamin behandelten Dünnschichtchromatographieplatte; Kaliumcarbonat enthaltendes, 7 : 3 Chloroform-Aceton-Gemisch).

Ms: $M^+$ 384 (5), m/e 383 (6), 313 (8), 238 (12), 222 (9), 207 (5), 196 (12), 158 (11), 143 (17), 123 (12), 121 (10), 115 (30), 111 (21), 99 (14), 95 (20), 91 (12), 83 (23), 81 (22), 69 (41), 55 (100).

Beispiel 3

Herstellung des 13-Oxa-13,14-dihydro-$PGI_2$-Natriumsalzes

Einer Lösung von 27 mg 13-Oxa-13,14-dihydro-$PGI_2$-methylester in 1,5 ml Methanol wird eine Lösung von 30 mg Natriumhydroxyd und 0,15 ml Wasser zugegeben, worauf das Reaktionsgemisch bei Raumtemperatur 15 Stunden gerührt wird. Nach der Lyophilisierung der Lösung werden 55 mg einer weißen Substanz erhalten, die 27,6 mg der im Titel genannten Verbindung enthält.

Beispiel 4

Herstellung des 15-Methyl-13-oxa-13,14-dihydro-$PGI_2$-Natriumsalzes und des 15-Epi-15-methyl-13-oxa-13,14-dihydro-$PGI_2$Natriumsalzes

72 mg des nach Beispiel 2 hergestellten, aus 15-Methyl-13-oxa-13,14-dihydro-$PGI_2$-methylester und 15-Epi-15-methyl-13-oxa-13,14-dihydro-$PGI_2$-methylester bestehenden Gemisches werden in 3,6 ml Methanol gelöst, worauf man eine Lösung von 74 mg Natriumhydroxyd und 0,40 ml Wasser zusetzt. Das erhaltene Reaktionsgemisch wird 16 Stunden bei Raumtemperatur gerührt. Nach der Lyophilisierung der Lösung werden 140 mg einer weißen Substanz erhalten, die 73,4 mg 15-Methyl-13-oxa-13,14-dihydro-$PGI_2$-Natriumsalz und 15-Epi-15-methyl-13-oxa-13,14-dihydro-$PGI_2$-Natriumsalz enthält.

0063337

1

## Patentansprüche

1. Optisch aktive und racemische 13-Oxa-prostacyclin-Derivate der allgemeinen Formel I

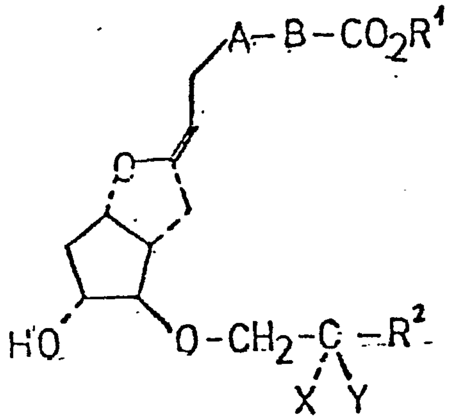

(I) ,

worin

$R^1$ Wasserstoff, ein pharmazeutisch verträgliches Kation oder geradkettiges oder verzweigtes $C_{1-5}$-Alkyl,

$R^2$ geradkettiges oder verzweigtes $C_{4-9}$-Alkyl oder monosubstituiertes Phenyloxymethyl,

-A-B- $-CH_2-CH_2-$, E- oder Z- -CH=CH- oder -C≡C-,

eines der Symbole X und Y Wasserstoff, Methyl oder Äthyl und das andere Hydroxy, Tetrahydropyranyloxy, 1-Äthoxy-äthoxy oder Tri-$C_{1-5}$-alkylsilyloxy bedeuten oder

X und Y gemeinsam $-O-CH_2-CH_2-O-$ bilden.

2. Methylester von
13-Oxa-13,14-dihydro-$PGI_2$,
15-Methyl-13-oxa-13,14-dihydro-$PGI_2$ oder
15-Epi-15-methyl-13-oxa-13,14-dihydro-$PGI_2$.

3. Natriumsalz von
13-Oxa-13,14-dihydro-$PGI_2$,

15-Methyl-13-oxa-13,14-dihydro-$PGI_2$ oder 15-Epi-15-methyl-13-oxa-13,14-dihydro-$PGI_2$.

4. Verfahren zur Herstellung von optisch aktiven und racemischen 13-Oxa-prostacyclin-Derivaten der allgemeinen Formel I

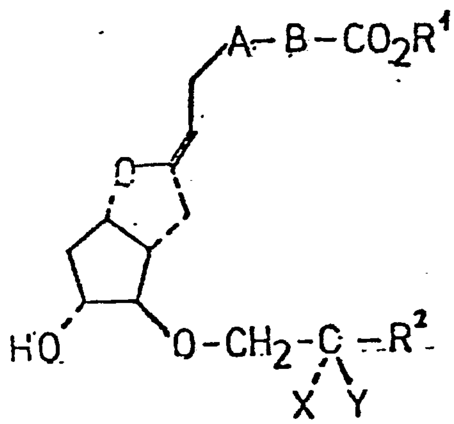

(I) ,

worin

$R^1$ Wasserstoff, ein pharmazeutisch verträgliches Kation oder geradkettiges oder verzweigtes $C_{1-5}$-Alkyl,

$R^2$ geradkettiges oder verzweigtes $C_{4-9}$-Alkyl oder monosubstituiertes Phenyloxymethyl,

-A-B- $-CH_2-CH_2-$, E- oder Z- -CH=CH- oder -C≡C-,

eines der Symbole X und Y Wasserstoff, Methyl oder Äthyl und das andere Hydroxy, Tetrahydropyranyloxy, 1-Äthoxy-äthoxy oder Tri-$C_{1-5}$-alkylsilyloxy bedeuten oder

X und Y gemeinsam $-O-CH_2-CH_2-O-$ bilden,

dadurch gekennzeichnet, daß man ein Epoxyderivat der allgemeinen Formel IV

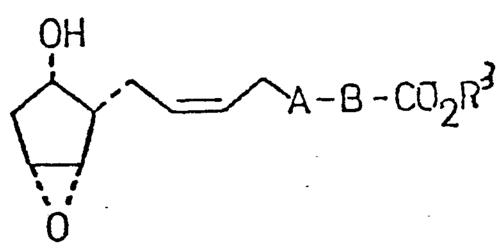

(IV),

worin

-A-B- die oben angegebene Bedeutung haben und

$R^3$ Wasserstoff oder $C_{1-5}$-Alkyl ist,

in wasserfreiem Medium mit einem aus einem primärem Alkohol der Formel V

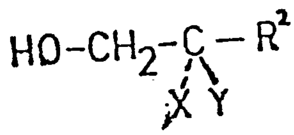

(V) ,

worin

X, Y und $R^2$ die oben angegebene Bedeutung haben,

gebildeten Alkoholat umsetzt, das erhaltene 13-Oxa-prostaglandin-Derivat der allgemeinen Formel III

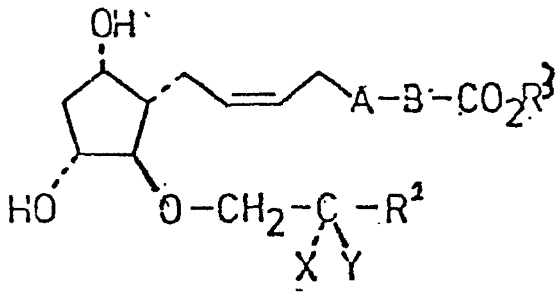

(III) ,

worin

-A-B-, X, Y, $R^2$ und $R^3$ die oben angegebene Bedeutung haben,

in Gegenwart einer schwachen Base mit einem Halogenierungsmittel behandelt, das erhaltene 5-Halogen-$PGI_1$-Derivat der allgemeinen Formel II

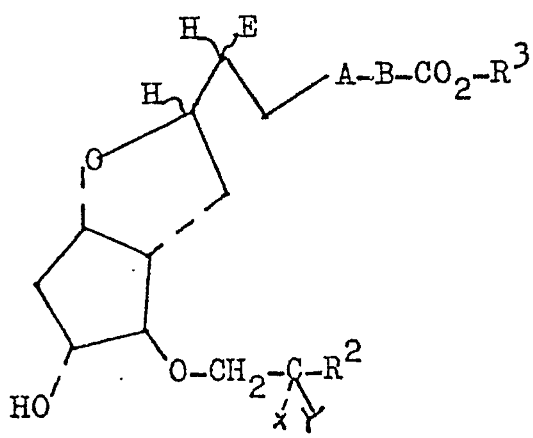

(II),

worin
-A-B-, X, Y, $R^2$ und $R^3$ die oben angegebene Bedeutung haben und E Halogen ist - falls E Brom ist, nach Abtrennung des Endo-Isomers bzw. falls E Jod ist, in Form des Isomergemisches - mit einer organischen oder anorganischen Base, in einem wasserfreien aprotischen Lösungsmittel oder in einem Alkanol bei einer Temperatur zwischen 25 und 150°C behandelt, und erwünschtenfalls die Substituenten der so erhaltenen Verbindung der allgemeinen Formel I in an sich bekannter Weise in andere Substituenten der Verbindungen der allgemeinen Formel I überführt.

5. Verfahren nach Anspruch 4, dadurch gekennzeichnet, daß man als Halogenierungsmittel ein Jodierungsmittel, vorzugsweise elementares Jod, verwendet.

6. Verfahren nach Anspruch 4 oder 5, dadurch gekennzeichnet, daß man die Dehydrohalogenierung mit einer organischen Base, vorzugsweise 1,5-Diazabicyclo-[4.3.0]non-5-en, durchführt.

7. Verfahren nach Anspruch 4, 5 oder 6, dadurch gekennzeichnet, daß man die Dehydrohalogenierung in Toluol beim Siedepunkt des Reaktionsgemisches durchführt.

8. Verfahren nach Anspruch 4,5,6 oder 7, dadurch gekennzeichnet, daß man bei der Halogenierung als schwache Base ein Alkalimetallcarbonat oder Alkalimetallhydroxyd verwendet.

9. Verfahren nach Anspruch 4,5,6,7 oder 8, dadurch gekennzeichnet, daß man die Halogenierung in einem mäßig polaren aprotischen Lösungsmittel durchführt.

10. Pharmazeutische Präparate, die als Wirkstoff ein oder mehrere der 13-Oxa-prostacyclin-Derivate der Ansprüche 1 bis 3 enthalten.

0063337

Europäisches Patentamt

# EUROPÄISCHER RECHERCHENBERICHT

Nummer der Anmeldung

EP 82 10 3072

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| | Keine Entgegenhaltungen.<br>----- | | C 07 D 307/935<br>A 61 K 31/34 |
| | | | RECHERCHIERTE SACHGEBIETE (Int. Cl. 3) |
| | | | C 07 D 307/00 |

Der vorliegende Recherchenbericht wurde für alle Patentansprüche erstellt.

| Recherchenort | Abschlußdatum der Recherche | Prüfer |
|---|---|---|
| DEN HAAG | 16-07-1982 | BERTE M.J. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument